# EUROPÄISCHE PATENTSCHRIFT

(11) **EP 0 789 580 B1**
(45) Veröffentlichungstag und Bekanntmachung des Hinweises auf die Patenterteilung: **05.06.2002**
(21) Anmeldenummer: 95925707.2
(22) Anmeldetag: 19.07.1995
(51) Int. Cl.: A61K 38/13, A61K 47/14, A61K 47/10, A61K 9/48

(54) **NEUE ZUBEREITUNGSFORMEN DES CYCLOSPORINS ZUR ORALEN APPLIKATION MIT EINFACHER ZUSAMMENSETZUNG UND HOHER BIOVERFÜGBARKEIT UND VERFAHREN ZU DEREN HERSTELLUNG**
NOVEL CYCLOSPORINE PREPARATION FORMS FOR ORAL ADMINISTRATION OF SIMPLE COMPOSITION AND HIGH BIO-AVAILABILITY, AND PROCESS FOR PRODUCING THEM
NOUVELLES FORMES DE PREPARATIONS DE CYCYLOSPORINES POUR ADMINISTRATION PAR VOIE ORALE, DE COMPOSITION SIMPLE ET HAUTE BIODISPONIBILITE, ET LEUR PROCEDE DE PRODUCTION

(30) Priorität: 03.11.1994 DE 4438861
(43) Veröffentlichungstag der Anmeldung: 20.08.1997
(73) Patentinhaber: Novartis AG, 4056 Basel (CH); Novartis-Erfindungen Verwaltungsgesellschaft m.b.H., 1235 Wien (AT)
(72) Erfinder: OLBRICH, Matthias, 01445 Radebeul (DE); PÖTTER, Heinrich, 01445 Radebeul (DE)
(86) Internationale Anmeldenummer: DE9500951
(87) Internationale Veröffentlichungsnummer: WO9614079

(56) Entgegenhaltungen:
- WO-A-95/22982
- GB-A- 2 221 157

## Beschreibung

Die Erfindung betrifft Cyclosporin, insbesondere Cyclosporin A enthaltende flüssige Zubereitungsformen zur oralen Applikation.

Cyclosporine sind neutrale zyklische Peptide, die mikrobiell hergestellt werden. Wichtigster Vertreter der Cyclosporine ist Cyclosporin A, das in der Transplantationsmedizin zur Unterdrückung der Organabstoßung und bei der Knochenmarkstransplantation eingesetzt wird.
Cyclosporin A, seine mikrobiologische Herstellung sowie seine Isolierung und Reinigung bis zu einem amorphen, farblosen Pulver ist aus der DE-PS 24 55 859 an sich bekannt .

Zunehmend findet Cyclosporin A auch Eingang in die Behandlung von Autoimmunerkrankungen, wie Psoriasis, Uveitis, nephrotisches Syndrom und andere.

Entzündungshemmende und antiparasitische Eigenschaften sind für die Cyclosporine beschrieben.

Aufgrund des hydrophoben Charakters von Cyclosporin ist es schwierig pharmazeutische Zubereitungen herzustellen, die zu einer hohen Bioverfügbarkeit des Wirkstoffes führen. Insbesondere zeigen die bekannten Darreichungsformen eine sehr breite inter- und intraindividuelle Variabilität der pharmakokinetischen Parameter. Bei gleicher Dosierung variiert der Cyclosporin-Blutspiegel von Patient zu Patient um bis zu 50 %. Selbst bei ein und dem selben Patienten schwankt die Resorbtion beträchtlich. Die immunsuppressive Therapie ist jedoch auf ein sehr enges therapeutisches Fenster zwischen dosisabhängigen Nebenwirkungen und Abstoßung des transplantierten Organ angewiesen.

Schlechte Bioverfügbarkeiten sind insbesondere auf die schlechte Löslichkeit des Cyclosporins bei der Mischung der Cyclosporine in Darreichungsformen mit Wasser zurückzuführen.

Es hat daher nicht an äußerst zahlreichen Versuchen gefehlt, diese galenischen Probleme zu lösen.
Bekannte, kommerziell verfügbare Darreichungsformen setzen folglich komplizierte Systeme aus lipophilen und hydrophilen Lösungsmitteln sowie lösungsvermittelnden Detergenzien ein, mit denen Cyclosporine in Lösung gebracht werden und in wäßrigen Systemen in Lösung gehalten werden sollen. Sie bestehen aus zumindest 4 Bestandteilen nämlich Wirkstoff, pflanzliches Öl, Ethanol und einem Tensid.

Aus US Patent 4,388,307 ist die Verwendung von Oel und Ethanol als Trägermedium in Verbindung mit Co-Lösungsmitteln bekannt. In Anlehnung an dieses Patent enthalten handelsübliche Trinklösungen von Ciclosporin Olivenöl, Ethanol und als oberflächenaktiven Stoff Labrafil ®. Diese Rezeptur bringt jedoch Probleme mit sich. Öle und oberflächenaktive Trägerstoffe haben oft einen unangenehmen Geruch und/ oder Geschmack. Außerdem neigen Öle mit ungesättigten Fettsäuren zum ranzig werden.
Zum zweiten ist in Rezepturen mit Ölen ein relativ hoher Gehalt an Ethanol erforderlich. Dieser hohe Ethanolgehalt bereitet jedoch Schwierigkeiten bei der Verabreichung der Präparate an Kinder und bringt Lagerprobleme mit sich.

Bei der Abfüllung in Kapseln muß zwecks Verdunstungsschutz ein erhöhter Aufwand bei der Konfektionierung durch Verpackung in Aluminiumblister betrieben werden.

Neuere Darreichungsformen gemäß Patent GB 2 222 770 beinhalten Lösungswege durch die Erzeugung von Mikroemulsionen. Diese Systeme bestehen aus 4 bis 6 Komponenten, die ein kompliziertes System aus Wirkstoff, lipophiler, hydrophiler Phase und einem oberflächenaktivem Stoff bilden. Systeme dieser Art beinhalten ein erhöhtes Risiko der Kreuzreaktion sowie das Risiko, daß einer der verwendeten Stoffe vom Patienten nicht vertragen wird.

Aus der DE-PS 39 24 207 ist ein Verfahren zur Herstellung peroral applizierbarer stabiler wässriger Injektionslösungen zur intravenösen Verabreichung bekannt, wonach
a)1 Gewichtsteil Cyclosporin
b) 8-13 Gewichtsteile von 1 oder mehr Monoester einer bzw. von gesättigten Hydroxyfettsäuren mit Polyethylenglykol und
c) 1-3 Gewichtsteilen von 1 oder mehr 1-und /oder mehrwertigen Alkoholen vermischt werden.
Oral anwendbare Arzneiformen sind in diesem Patent nicht hergestellt und untersucht worden. Wenn man versucht, diese Rezepturen mit Wasser zu verdünnen kommt es zu Ausfällungen des Cyclosporins und damit zu einer wesentlichen Einschränkung der Bioverfügbarkeit.

Alle kommerziell erhältlichen Darreichungsformen enthalten ölige, lipophile Bestandteile (Maisöl, Kernöl, Maisöl-mono-di-tri-glyceride) und ein bzw. mehrere Detergenzien sowie ein- oder mehrwertige Alkohole.

Aus der DE-OS 38 43 054 ist zu entnehmen, daß sich orthorhombisch kristalline Formen wie CY-A/X-II und vor allem CY-A/X-III besonders gut zur Herstellung galenischer Formen eignen. Diese Formulierungen sollen Ciclosporin in stabiler und feinteiliger Form enthalten und/oder über eine bessere Stabilität verfügen oder günstigere Freisetzungscharakteristiken aufweisen. Bevorzugt kommen diese Formen zur topisch dermalen oder topisch opththalmischen Anwendung. Das beschriebene Herstellungsverfahren für die solvatfreie orthorhombische Kristallform unter Verwendung von Ultraschall ist im technischen Maßstab schwer zu realisieren.
Gleichfalls wird dargestellt, daß Ciclosporin in amorpher Form weniger gut für die Herstellung von Darreichungsformen geeignet ist.

Die dargestellte Problematik wurde dadurch erfindungsgemäß gelöst, daß überraschenderweise festgestellt wurde, daß in Darreichungsformen des Cyclosporins zur oralen Applikation mit einfacher Zusammensetzung und hoher

Bioverfügbarkeit in Form von Trinklösung oder Kapseln enthaltend:
a) 0,5 bis 2, bevorzugt 1 Gewichtsteile von einem oder mehreren amorphe(n) Cyclosporin(en) als Wirkstoff
b) 6 bis 9, bevorzugt 7,5 Gewichtsteile ein oder mehrere Polyethylenglykol-Monoester von gesättigten C 10 bis C22 Hydroxyfettsäuren, vorzugsweise SOLUTOL® HS15
c) 1-3, bevorzugt 2 Gewichtsteile ein oder mehrere ein- oder mehrwertige Alkohole als Co-Lösungsmittel vorzugsweise Ethanol und Propylenglykol die Löslichkeit des (der) Cyclosporin(e)s insbesondere in Verdünnungen mit Wasser bei Einhaltung dieser speziellen Mengenverhältnisse wesentlich erhöht ist.

Das war nicht normalerweise anzunehmen, da vergleichbare Darreichungsformen Polyethylenglycolester von Fettsäuren nur als zusätzlichen Lösungsvermittler zwischen hydrophober und hydrophiler Phase einsetzen.

Es war daher um so überraschender, daß eine derartige Rezeptur eine Bioäquivalenz zu kommerziellen Produkten (s.o.) zeigte.

Insbesondere war es nicht vorauszusehen, daß eine so einfache Rezeptur ohne lipophile Komponente derartig hohe Bloverfügbarkeiten erreichen konnte.

Es wurde ferner gefunden, daß gerade der Einsatz von amorphem Cyclosporin in einer oralen Applikationsform zu besonders guten Lösungseigenschaften in Rezepturen mit einem Gehalt an Cyclosporin > 5 % führt, die auch in Verdünnungen mit Wasser als stabile, klare Lösung erhalten bleiben.

Gegenstand der Erfindung sind daher orale Darreichungsformen, welche als Trinklösung oder abgefüllt in Kapseln folgende Bestandteile in folgenden Mengenverhältnissen enthalten:
a) 0,5-2 Gewichtsteile bevorzugt 1 Gewichtsteil ein oder mehrere Cyclosporine, insbesondere Cyclosporin A oder G , welches in amorpher Form eingesetzt wird
b) 6 - 9 Gewichtsteile, bevorzugt 7,5 Gewichtsteile ein odere mehrere Polyethylenglycol-Monoester mit im Molekül gebundenen gesättigten C10 bis C22 -Hydroxyfettsäureanteilen, insbesondere SOLUTOL ® HS 15
c) 1 bis 3 Gewichtsteile, vorzugsweise 2 Gewichtsteile ein oder mehrere einoder mehrwertige Alkohole als Co-Lösungsmittel vorzugsweise Ethanol und Propylenglykol.

Bei dem ebenfalls erfindungsgemäßen Herstellungsverfahren ist darauf zu achten, daß die Mengenverhältnisse eingehalten werden und daß das Cyclosporin unter ständigem Rühren bei Raumtemperatur zunächst vollständig in Ethanol gelöst wird und anschließend ebenfalls unter ständigem Rühren und auch bei Raumtemperatur die Zugabe von Propylenglykol und Solutol ® HS 15 erfolgt. Die nach diesem Verfahren hergestellten Lösungen enthalten 100mg/ml Wirkstoff.
DieKonfektionierung als Trinklösung oder Kapseln erfolgt in bekannter Weise z. B. in Kapseln zu je 100mg, 50 mg oder 25 mg Wirkstoff.

Die Herstellung der erfindungsgemäßen Zusammensetzung wird in den nachfolgenden Beispielen näher erläutert :

### Beispiel 1

100 g amorphes Cyclosporin A werden unter Rühren bei Raumtemperatur in 127 ml Ethanol gelöst. Anschließend werden unter weiterem Rühren bei Raumtemperatur 96 ml Propylenglykol zugegeben. Nach klarem Lösen des Cyclosporin A werden 750 g Solutol® HS 15 bei weiterem Rühren zugegeben. Es entsteht eine klare, viskose Lösung mit einem Gehalt von 100 mg/ml Cyclosporin A.

### Beispiel 2

Eine nach Beispiel 1 hergestellte Cyclosporin-A-Lösung wird im Verhältnis 1 : 40 mit Wasser verdünnt. Die erhaltene Lösung bleibt über mehrere Monate klar und stabil.

## Patentansprüche

1. Zubereitungsformen des Cyclosporins zur oralen Applikation in Form von Trinklösung oder Kapseln enthaltend:
a) 0.5 bis 2, bevorzugt 1 Gewichtsteil ein oder mehrere amorphe(s) Cyclosporin(e) als Wirkstoff
b) 6 bis 9, bevorzugt 7.5 Gewichtsteile ein oder mehrere Polyethylenglykol-Monoester von gesättigten C10 - C22 Hydroxyfettsäuren, vorzugsweise Polyethylenglykol-660-12-(hydroxy)-stearat
c) 1 bis 3, bevorzugt 2 Gewichtsteile ein oder mehrere ein- oder mehrwertige Alkohole als Co-Lösungsmittel vorzugsweise Ethanol und Propylenglykol.

2. Zubereitungsform nach Anspruch 1, worin als Wirkstoff Cyclosporin A oder Cyclosporin G in amorpher Form eingesetzt werden.

3. Zubereitungsform nach Anspruch 1 oder 2 enthaltend Ethanol und/oder Propylenglykol als ein- oder mehrwertigen Alkohol.

4. Zubereitungsform nach einem der Ansprüche 1 bis 3 in Form von Kapseln.

5. Verfahren zu Herstellung der Zubereitungsformen des Cyclosporins zur oralen Applikation in Form von Trinklösung oder Kapseln nach einem der Ansprüche 1 bis 3 in dem zunächst unter Rühren bei Raumtemperatur
a) per 100 g amorphes Cyclosporin, insbesondere Cyclosporin A in 127 ml Ethanol gelöst werden
b) ebenfalls unter Rühren und bei Raumtemperatur die Zugabe von 96 ml Propylenglykol erfolgt und abschließend ebenfalls unter Rühren bei Raumtemperatur
c) 750 g Polyethylenglykol-660-12-(hydroxy)-stearat zugefügt werden, wobei eine klare, viskose Lösung des Cyclosporin A entsteht, die in an sich bekannter Weise als Trinklösung oder Kapseln abgefüllt wird.

## Claims

1. Cyclosporin formulations for oral administration in the form of a drinking solution or capsules containing:
a) 0.5 to 2, preferably 1, part by weight of one or more amorphous cyclosporin(s) as active ingredient,
b) 6 to 9, preferably 7.5, parts by weight of one or more polyethylene glycol monoesters of saturated C10 - C22 hydroxy fatty acids, preferably polyethylene glycol-660-12-(hydroxy)-stearate,
c) 1 to 3, preferably 2, parts by weight of one or more mono- or polyvalent alcohols as cosolvents, preferably ethanol and propylene glycol.

2. Formulation according to claim 1, in which cyclosporin A or cyclosporin G is used in amorphous form as active ingredient.

3. Formulation according to claim 1 or 2, containing ethanol and/or propylene glycol as the mono- or polyvalent alcohol.

4. Formulation according to one of claims 1 to 3 in the form of capsules.

5. Process for the production of the cyclosporin formulations for oral administration in the form of a drinking solution or capsules according to one of claims 1 to 3, in which, initially while stirring at room temperature,
a) 100 g of amorphous cyclosporin, especially cyclosporin A, are dissolved in 127 ml of ethanol,
b) likewise while stirring and at room temperature, 96 ml of propylene glycol are added, and subsequently, likewise while stirring at room temperature,
c) 750 g of polyethylene glycol-660-12-(hydroxy)-stearate are added, resulting in a clear, viscous solution of the cyclosporin A, which is, in a manner known *per* se, bottled as a drinking solution or packed into capsules.

## Revendications

1. Formes de préparation de la cyclosporine en vue de l'application orale en solution buvable ou en gélules contenant :
a) 0,5 à 2, de préférence 1 partie en poids, d'une ou plusieurs cyclosporine(s) amorphe(s) comme substance(s) active(s) ;
b) 6 à 9, de préférence 7,5, parties en poids d'un ou plusieurs monoesters de polyéthylèneglycol d'hydroxyacides gras saturés en C₁₀ à C₂₂ saturés, de préférence de 12-(hydroxy)-stéarate de polyéthylèneglycol-660 ;
c) 1 à 3, de préférence 2, parties en poids, d'un ou plusieurs alcools mono- ou polyvalents comme co-solvants, de préférence l'éthanol ou le propylèneglycol.

2. Forme de préparation selon la revendication 1, dans laquelle on utilise comme substance active la cyclosporine A ou la cyclosporine G sous forme amorphe.

3. Forme de préparation selon la revendication 1 ou 2 contenant de l'éthanol et/ou du propylèneglycol comme alcool mono- ou polyvalent.

4. Forme de préparation selon l'une des revendications 1 à 3 sous forme de gélules.

5. Procédé de préparation des formes de préparation de la cyclosporine en vue de l'application orale sous forme de solution buvable ou de gélules selon l'une des revendications 1 à 3, dans lequel tout d'abord, tout en agitant à la température ambiante,
a) on dissout pour 100 g de cyclosporine amorphe, en particulier de cyclosporine A, dans 127 ml d'éthanol,
b) également tout en agitant, et à la température ambiante, on procède à l'addition de 96 ml de propylèneglycol, et enfin, également tout en agitant à la température ambiante,
c) on ajoute 750 g de 12-(hydroxy)-stéarate de polyéthylèneglycol-660, et il apparaît une solution claire, visqueuse, de cyclosporine A, que l'on conditionne d'une façon connue en soi comme solution buvable ou en gélules.
